Europäisches Patentamt

European Patent Office

Office européen des brevets

(19)

(11) Publication number: **0 159 289 B1**

## EUROPEAN PATENT SPECIFICATION

(12)

(45) Date of publication of patent specification: **06.05.92**

(51) Int. Cl.⁵: **C12P  21/00**, A61K 37/02, //(C12P21/00,C12R1:91)

(21) Application number: **85810114.0**

(22) Date of filing: **15.03.85**

(54) Immunosuppressant factor.

(30) Priority: **23.03.84 EP 84810140**
**23.11.84 GB 8429679**

(43) Date of publication of application:
**23.10.85 Bulletin  85/43**

(45) Publication of the grant of the patent:
**06.05.92 Bulletin  92/19**

(84) Designated Contracting States:
**AT BE CH DE FR GB IT LI LU NL SE**

(56) References cited:

NEUROLOGY, vol. 34, no. 3, supplement 1,
March 1984, 36th ANNUAL MEETING PRO-
GRAM, 8th-14th April 1984, Boston, Massa-
chusetts, page 184, The American Academy
of Neurology, US; A. FONTANA et al.:
"Immunodeficiency in glioblastoma patients:
glioblastoma cells release factors inhibiting
interleukin-2-mediated effects on T cells"

(73) Proprietor: **SANDOZ AG**
**Lichtstrasse 35**
**CH-4002 Basel(CH)**

(84) Designated Contracting States:
**BE CH FR GB IT LI LU NL SE**

(73) Proprietor: **SANDOZ-PATENT-GMBH**
**Humboldtstrasse 3**
**W-7850 Lörrach(DE)**

(84) Designated Contracting States:
**DE**

(73) Proprietor: **SANDOZ-ERFINDUNGEN Verwal-
tungsgesellschaft m.b.H.**
**Brunner Strasse 59**
**A-1235 Wien(AT)**

(84) Designated Contracting States:
**AT**

(72) Inventor: **Fontana, Adriano Section of Clinical
Immunology**
**Dept. of Internal Medicine University Hos-
pital**
**Haldeliweg 4 CH-8044 Zurich(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(-/2.18/2.0)

THE JOURNAL OF IMMUNOLOGY, vol. 129, no. 6, December 1982, pages 2413-2419, The American Association of Immunologists, US; A. FONTANA et al.: "Production of prostaglandin E and an interleukin-1 like factor by cultured astrocytes and C6 glioma cells"

THE JOURNAL OF IMMUNOLOGY, vol. 132, no. 4, April 1984, pages 1837-1844, The American Association of Immunologists, US; A. FONTANA et al.: "Glioblastoma cells release interleukin 1 and factors inhibiting interleukin 2-mediated effects"

## Description

The present invention is concerned with an immunomodulatory factor.

It is more particularly concerned with an immunosuppressant factor isolated from human glioblastoma cells.

The immunosuppressant factor has influence on T-cells.

The role of T-cells in cell-mediated immunity (e.g. by cooperation with B-lymphocytes) is well established. Substances which are capable of inhibiting T-cell mechanisms involved in the generation of antibodies or in lysis of target cells would be used to suppress or reduce the body's immune response. Such substances could thus be employed e.g. in connection with transplants to prevent rejection and also in connection with the treatment of diseases characterised by an auto-immune response in the body.

We have now found that cultured human glioblastoma cells secrete a factor that inhibits IL-2 dependent T-cell mechanisms.

This factor has inhibitory effects and has the following features:

the factor

aa) inhibits the incorporation of tritiated thymidine into murine thymocytes stimulated with Concanavalin A or phytohaemaglutinin in the presence of IL-2;

bb) inhibits the proliferation of IL-2 dependent T cell clones;

cc) suppresses the growth of neuroblasts but not fibroblasts;

dd) inhibits the generation of cytotoxic T cells in the allogenic mixed lymphocyte reaction;

ee) inhibits the proliferation of hapten-specific cytotoxic T cells in the presence of haptenated stimulator;

ff) inhibits the proliferative response of thymocytes to concanavalin A and

hh) is sensitive to tryptic proteolysis.

The immunosuppressant factor is capable of inhibiting T-cell mechanisms particularly those which are Interleukin 2 (IL-2) dependent.

This factor has an inhibitory effect on IL-2 effects on thymocytes in the presence of lectins and on the induction of alloreactive cytotoxic T-cells in mixed lymphocyte cultures (MLC). IL-2 does not substitute for the inhibitor effect of the suppressor factor on the induction of the MLC. It further inhibits the growth of neuroblasts but not of fibroblasts. The glioblastoma cell derived suppressor factor was also found to inhibit the lectin response of human peripheral blood mononuclear cells isolated from blood donors. However, the inhibitory effect of the factor was maximal at sub-optimal lectin concentrations.

This factor can be obtained from the cystic fluid of glioblastoma tumors or from supernatant (SN) of cultured human glioblastoma cell lines and has an apparent approximate molecular weight of 97,000 and is also referred to herein as G-TsF.

Cultured human glioblastoma cells are characterised by ultrastructural features, especially the presence of filaments, and by biochemical markers, such as glial fibrillary acidic protein (GFA) and S-100 protein (S.C. Diserens et al, Acta Neuropathol. 53: 21; A. Pactau et al, Acta Neuropathol. 47: 71; A. Edstrom et al, Exp. Cell Res. 83: 426). In addition human glioma cells express neuroectodermal antigens shared with melanomas and neuroblastomas (S.N. Carrel et al, Acta Neuropathol., 57: 158), as well as Ia-like antigens and the common acute lymphoblastic leukemia antigen (CALLA), an antigen expressed on lymphoid cells from patients with the common form of acute lymphoblastic leukemia.

One immunoregulatory mediator, a suppressor factor, was detected in the supernatant of glioblastoma cells. This factor inhibits the proliferative response of T cells to Con A (concanavalin A) or to both PHA (Phytohemagglutinin) and IL-1/IL-2 (Interleukin 1/Interleukin 2) standards. The factor also blocks the proliferation of an H-2-restricted, hapten-specific T cell line that normally grows in the presence of IL-2 (Interleukin 2) and haptenated irradiated spleen cells. The glioblastoma-derived suppressor factor has no effect on IL-2 production by Con A-stimulated spleen cells and does not influence the growth of a thymoma cell line (EL 4 cells), which proliferates independently of IL-2. Taken together, these results indicate that the factor interferes with terminal events in the T cell activation cascade. The hypothesis that this glioblastoma-derived suppressor factor influences proliferative steps not necessarily involving direct direct IL-2 effects on T cells is based on the finding that the 97,000-m.w. factor does also inhibit the growth of neuroblasts, which grow independently of IL-2.

Recently, the presence of immunosuppressive factors in the conditioned medium of various human tumor cells was investigated (C.M. Renk et al, Cancer Immunol. Immunother. 11: 7). Out of 12 different tumors (three melanoma, two sarcoma, and seven carcinoma) the SN of only three tumors produced greater than 30 % inhibition of PHA-induced blastogenesis. However, as shown by dilution experiments the inhibitory effect was only visible up to 1/4 dilution of the SN; higher dilutions had no effect. When using the conditions to produce and to test the glioblastoma SN, the SN of two neuroblastoma, a melanoma, and a rhabdomyosarcoma had no effect on responsiveness of thymocytes to Con A. This supports the assumption that among nonlymphoid tumors glioblastoma cells are at the present time unique for their capacity to release immunosuppressive factors in vivo and in vitro.

Depression of immune responsiveness has been documented in patients with glioblastoma (W.H. Brooks et al, J. Exp. Med. 136: 1631; H.F. Young et al, Surg. Neurol. 5: 19). Impaired cell-mediated immunity in glioblastoma patients is indicated by depressed skin reactivity to common antigens, decreased ability to become sensitised to dinitrochlorobenzene, and decreased in vitro lymphocyte responsiveness to PHA or antigens. Factors released by the tumor cells may well account for the described T cell immune deficiency state. This idea is supported by the detection of inhibitory factors on T cell activation present in glioblastoma culture SN in vitro and in the cyst fluid of glioblastoma tumors in vivo. In addition the sera of glioblastoma patients contain a factor that inhibits the MLC and the PHA- and Con-A-induced lymphocyte proliferation in vitro. After removal of the glioblastoma by neurosurgery, suppressor activity in the sera disappeared.

Although it is well documented that in most glioblastoma patients, humoral immune responses to their tumors develop (M. Pfreundschuh et al, Proc. Natl. Acad. Sci. USA 75: 5122), there is little evidence of significant cellular anti-tumor immune responses (K.M.A. Sheik et al, Cancer Res. 39: 1733). In general, cytotoxic T cells have been implicated to contribute to the development of the cellular anti-tumor immune reactions (M.A. Cheever et al, J. Immunol. 126: 1318). The IL-2 dependency of the generation of CTL has been demonstrated by in vivo administration of highly purified IL-2 in concert with injections of tumor cells, which resulted in an augmented CTL and natural killer cell response in mice (S.H. Heffeneider et al, J. Immunol. 130: 222).

The observed blocking of the generation of CTL by the glioblastoma cell-derived 97.000 m.w. factor according to the invention may account at least in part for inefficient immunosurveillance against glioblastomas.

Impaired host immunocompetence may also emanate from lymphokine-induced production of mucopolysaccharide coats by glioblastoma cells that nonspecifically suppress the cellular immune response.

G-TsF is characterised by its activity spectrum as described above and in the examples hereinafter and its approximate molecular weight. Furthermore it is sensitive to tryptic proteolysis and had a peak of pI 4.6 on isoelectric focusing as hereinafter described.

The factor of the invention can be purified effectively by a method which comprises concentrating the supernatant of a human glioblastoma cell line by ultrafiltration and subjecting same to sequential chromatography on Blue Affigel, hydroxyapatite and gel filtration.

Purification can take place by various procedures such as conventionally used for the purification of cell factors. A particularly advantageous procedure involves successive chromatography of SN (optionally with pre-desalination) using first Blue-Affigel and then Hydroxyapatite followed by gel filtration e.g. on Ultrogel AcA54, or on Ultro Pac TSK G 3000 SWG. Other absorbents which can be used include Phenyl-Sepharose, DEAE-cellulose and Heparin affigel. Other methods include the use of suitable antibodies.

In addition to its relevance for modulation of T cell activation the conditioned media of the glioblastoma is of interest for its neuroblast growth inhibitory factor (NGIF)-like activity. NGIF is released by fetal rat glioblasts and exerts suppressor activities on neuroblasts but not on fibroblasts (Y. Sakazaki et al, Brain Res. 262: 125). In an analogous assay we also observed that the Sephacryl S-200 glioblastoma fractions, which inhibited T cell responsiveness to IL-2 and the growth of neuroblasts, did induce neurite outgrowth, but had no effect on fibroblasts.

The following examples illustrate the invention. The following abbreviations are employed:

IL 1, Interleukin 1; IL 2, Interleukin 2; NGIF, neuroblast growth inhibition factor; SN, supernatant; GFA, glial fibrillary acidic protein; DMEM, Dulbecco's modified Eagle's medium; ConA, concanavalin A; 2-ME, 2-mercaptoethanol; $^3$H-Tdr.[$^3$H]thymidine; PHA, phytohemagglutinin; OVA cells, ovalbumin specific T-cell line; CTL, cytotoxic T-lymphocytes; MLC mixed lymphocyte culture; LPS, Lipopolysaccharide; AED, N-iodoacetyl-N-[5-sulfonic-1-naphthyl]ethylene diamine; CH, cycloheximide; FCS, fetal calf serum; G-TsF, Glioblastoma derived T cell suppressor factor.

Example 1

Four human glioblastoma cell lines are established and maintained in mono-layer cultures by the method described in Diserens et.al., Acta Neuropathol. 53:21. Four cells lines used in the examples are designated C1-18; C1-229; 992 and 308 and were maintained for 72, 28, 8 and 16 months respectively.

4

Characterization of the glioblastoma cell lines revealed that cells from C1-229, 308 and 992 expressed Ia-like antigens on their surface, cells from 308 and 992 were positive for glial fibrillary acidic protein (GFA), and cells from C1-18, 308 and 992 expressed a neuroectodermal antigen identified by recently described monoclonal antibodies (Acta Neuropathol. 57:158 Carrell S., et.al.)

For production of SN the glioblastoma cells were plated at $1 \times 10^4$ to $1 \times 10^6$ cells/well (2.4 x 1.7 cm; Linbro Scientific) in culture medium consisting of Dulbecco's modified Eagle's medium (DMEM), 10% fetal calf serum (FCS), and 300 ug/ml L-glutamine. Twenty-four hours after plating the glioblastoma cells, the medium was replaced with 0.5 ml culture medium. The SN were collected 1 to 5 days later, centrifuged (2000 x G. 10 min) and ultrafiltrated by using a micropartition system with YMB membrane (MPS.1, Amicon). The material deposited after centrifugation (1500 x G. 15 min) on the membrane (m.w.> 10.000), was resuspended in RPMI 1640 to give the original volume and passed through 0.45-μm filters.

## Example 2 - Selection of absorbents

The following adsorbents were tested: Bio-Gel HTP (hydroxyapatite), Blue-Affigel, Heparin-Affigel (Bio-Rad, Richmond, CA); Phenyl-Sepharose, ConA-Sepharose, Protein A-Sepharose (Pharmacia, Uppsala, Sweden); DEAE-Cellulose, CM-Cellulose (Serva, Heidelberg, Germany). Each adsorbent was equilibrated in the appropriate adsorption buffer listed in Table I and the slurry was transferred to a disposable 5 ml-plastic column (Isolab, Akron, OH) to give 0.2 ml of settled volume. After closing the column at the bottom, 0.5 ml of a G-TsF standard (activity 100 units) was added and the slurry was kept in suspension overnight at 4°C on a rotating turn table. The column was then drained, and the effluent was desalted and assayed for protein content [Lowry et.al., J. Biol. Chem; 193: 265; 1951] and for G-TsF activity using the Neuro 2A assay (see below). The adsorbent was resuspended in the appropriate desorption buffer (Table IV), kept in suspension for 2 hours at 4°C, drained and G-TsF and protein content in the column effluent were determined as above. Overall recoveries were at least 80%.

## Example 3 - Purification of G-TsF (see Table V)

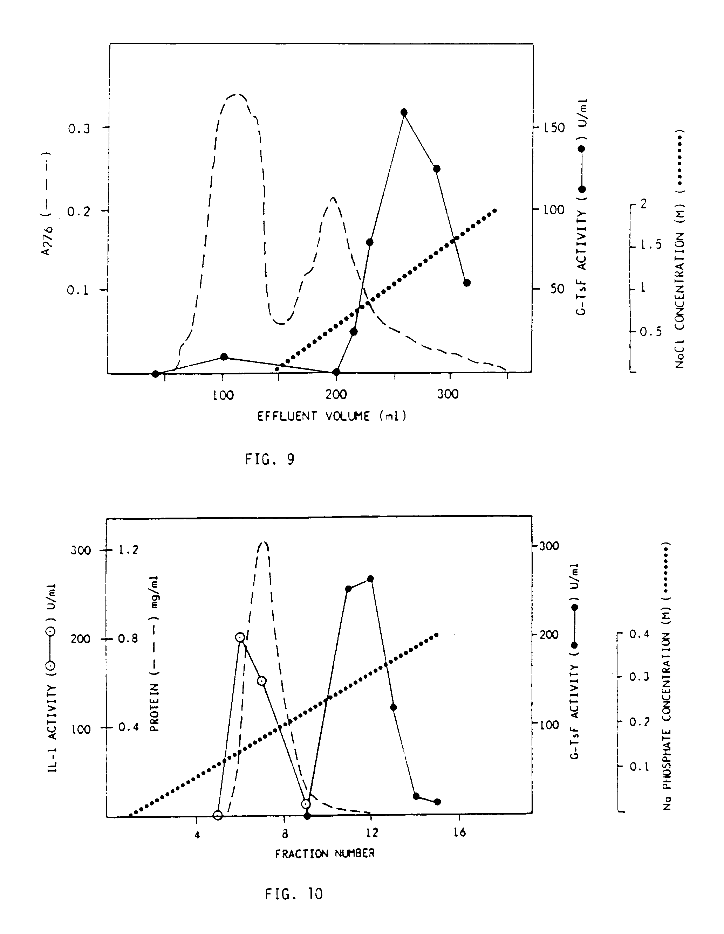

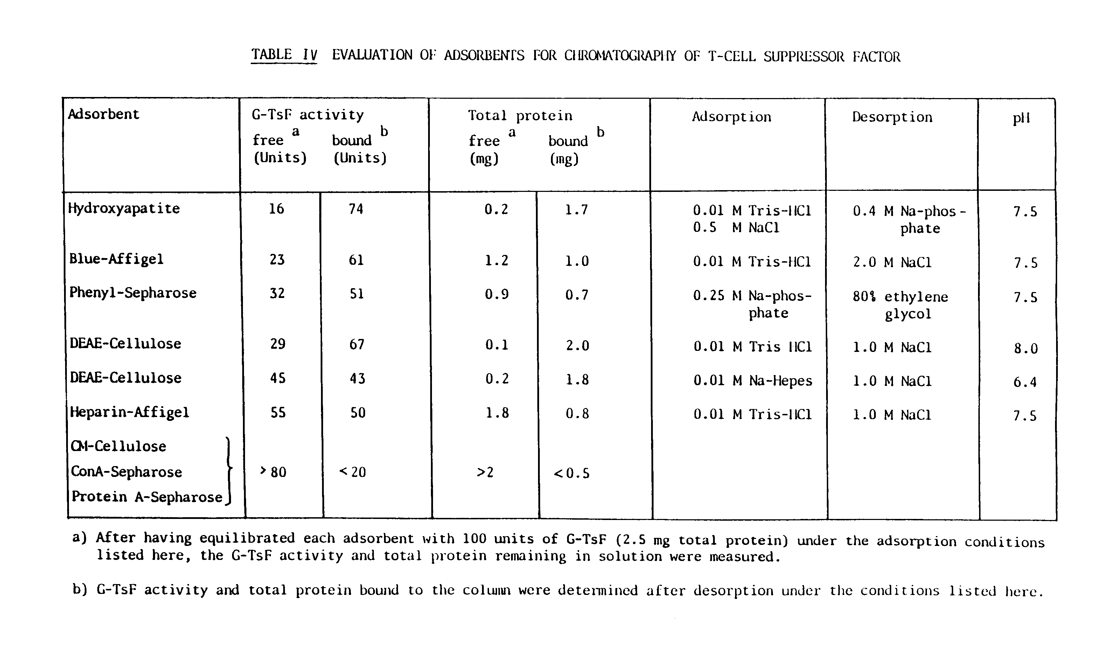

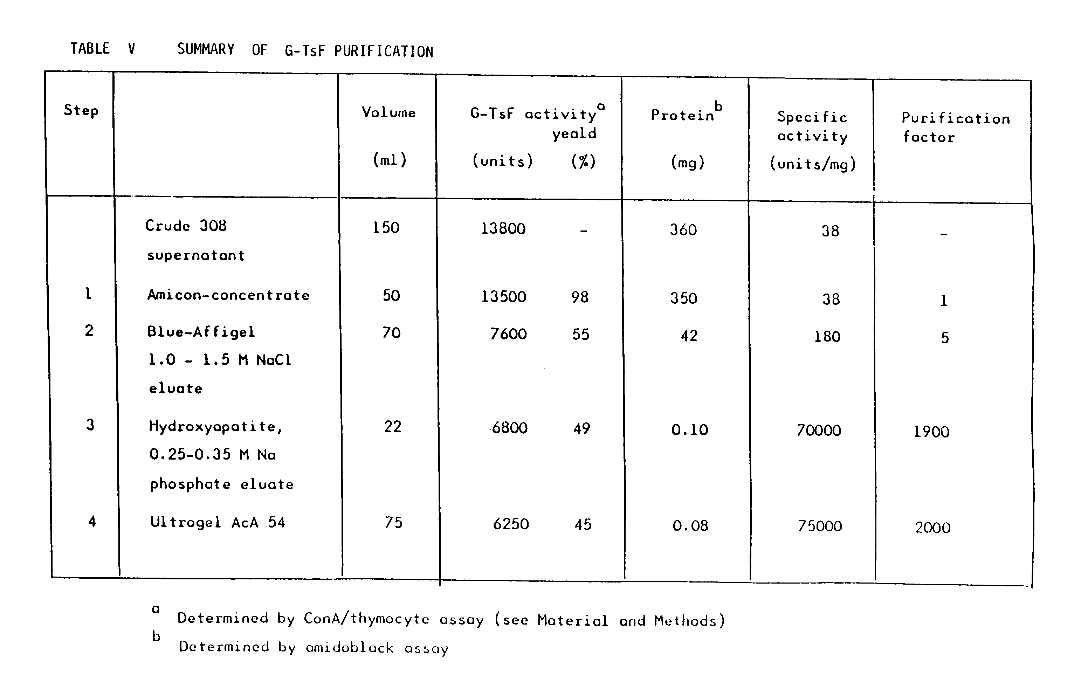

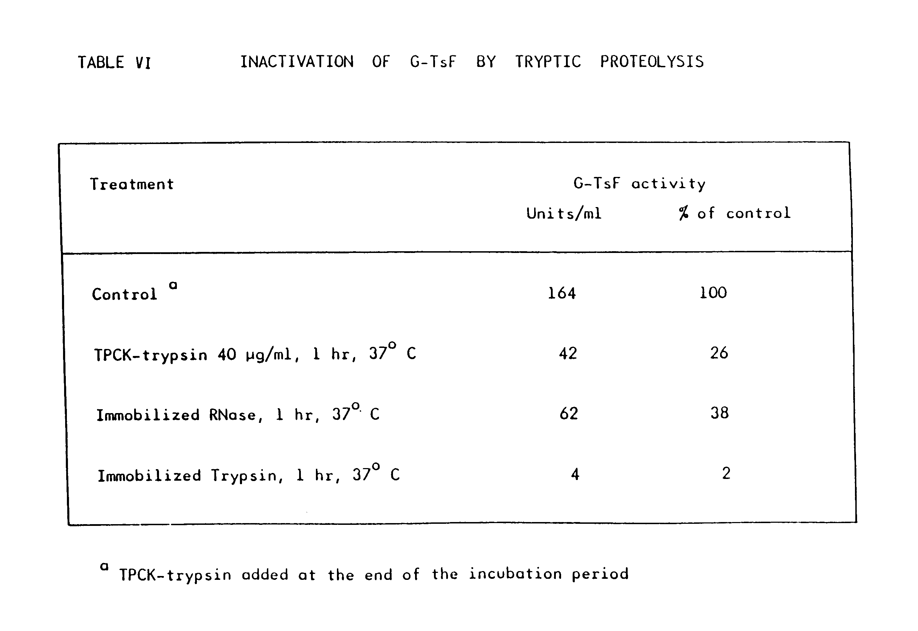

The 308 SN (150 ml) was concentrated 6-fold by ultrafiltration through a 43 mm YM10 membrane (Amicon, Danvers, MA) and then rediluted to a volume of 50 ml by adding 10 mM Tris-HCL, pH 7.5. The concentrate was applied at 4°C to a column of Blue-Affigel (1.6 x 35 cm; 70 ml bed volume) at a flow rate of 48 ml/hr. The column was eluted with a linear gradient (280 ml) of 2 M NaCl in 10 mM Tris-HCl, pH 7.5. The fractions containing G-TsF activity as determined on ConA stimulated thymocytes (70 ml; 1.0 - 1.5 M NaCl) (cf. Fig. 9) were pooled, diluted with 70 ml of 10 mM Na phosphate, pH 7.5, and applied at room temperature to a column of hydroxyapatite (Bio-Gel HTP; 0.8 x 28 cm; 14 ml) at a flow rate of 12 ml/hr. From here on, all fractions were collected in siliconized plastic tubes [Maniatis et.al., Molecular cloning. Cold Spring Harbor Laboratory, New York, p. 437]. The column was eluted with a linear gradient (105 ml) consisting of 10 mM Na phosphate, pH 7.5, 0.15 M NaCl (start buffer) and 0.5 M Na phosphate, pH 7.5 (limit buffer). Each of the fractions (7.5 ml) containing G-TsF activity (cf. Fig. 10) (0.25-0.35 Na phosphate) was applied in a separate run to a column of Ultrogel AcA 54 (LKB, Bromma, Sweden) (1,6 x 88 cm; 176 ml) which had been equilibrated at 4°C in 10 mM Tris-HCl, pH 7.5, 0,15 M NaCl and calibrated with Blue Dextran (v), bovine serum albumin (BSA), ovalbumin, and cytochrome c (Cyc). The column was eluted at 20 ml/hr with the same buffer, and the fractions containing G-TsF activity were pooled and stored at -20°C. Alternatively the fractions from the HTP column containing G-TsF activity were concentrated by ultrafiltration through a YM10 membrane and further purified on a preparative HPLC gel filtration column (TSK 3000 SWG LKB-Bromma). Protein concentrations were determined with the amidoblack assay [Schaffner, Weissmann Anal Biochem 56:514, 1973] or spectrophotometrically and salt concentration by conductivity.

## Example 4 - Isoelectric focusing

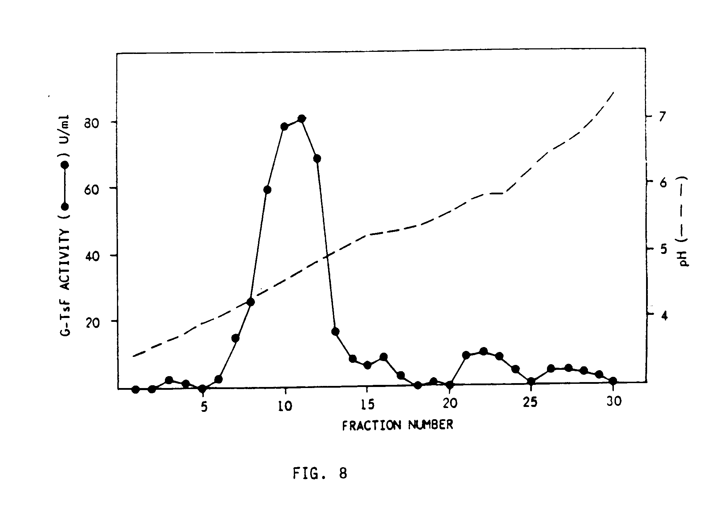

A sample of the G-TsF (60 ml) obtained after Example 3 (Table V step 4) was concentrated by ultrafiltration through an Amicon YM10 membrane for partial removal of salt which interferes with formation of the pH gradient. The G-TsF was then mixed with 85 ml Ultrodex (4 g preswollen in 100 ml $H_2O$), 2.5 ml Ampholines pH 3.5 - 5, and 2.5 ml Ampholines pH 5- 8 (all from LKB) at 10W and 1'2000 V for 16 hr. After the run, the gel was sliced into 30 fractions, and their pH was determined with a surface glass electrode. Each fraction was transferred to a disposal 5 ml-plastic column, and the proteins were eluted with 2 ml of 10 mM Tris-HCl, pH 7.5, 0.15 M NaCl, desalted in Sephadex G50 columns to remove Ampholines, and assayed for G-TsF activity using the ConA-thymocyte assay (see below). A peak was determined at $p^I$ 4.6. (cf. Fig. 8 ).

Example 5 - Tryptic proteolysis

Samples of G-TsF (0.2 ml) obtained after Example 3 (Table V step 3) were incubated for 1 hr at 370°C with 8 $\mu$g trypsin (Worthington; treated with L-1-tosylamido-phenylethyl chloromethyl ketone), added either at the beginning or - as a control - at the end of the incubation period. Additional samples were incubated with Sepharose-bound trypsin (0.2 ml settled gel; Worthington) or with an equivalent amount of RNase A coupled to Affigel 10 (Bio Rad). The reactions were stopped by using the desalting protocol described above and then testing for G-TsF activity in the ConA-thymocyte assay. The results are shown in Table VI. Parallel incubations with 125 I labelled factor indicated a loss of protein by non-specific adsorption to the Affigel 10, thus explaining the low recovery of G-TsF activity from the RNAase column

Example 6 : Thymocyte proliferation assay

The effect of glioblastoma SN on the thymocyte proliferative response to concanavalin A (Con A) was tested in the following way. Samples of 50 $\mu$l of SN at various dilutions were added to 6 x $10^5$ thymocytes from $C_3$H/HeJ mice: thymocytes were suspended in 150 ul of RPMI 1640 medium supplemented with 300 $\mu$g/ml L-glutamine. 1 x $10^{-5}$ M 2-mercaptoethanol (2-ME). and 5% FCS in flat-bottomed microtiter plates and incubated for 72 hours in the presence of Con A (1 $\mu$g/well). Sixteen hours before harvest 0.5 $\mu$Ci of $^3$H-Tdr.(5.0 Ci/mmol) was added per well.

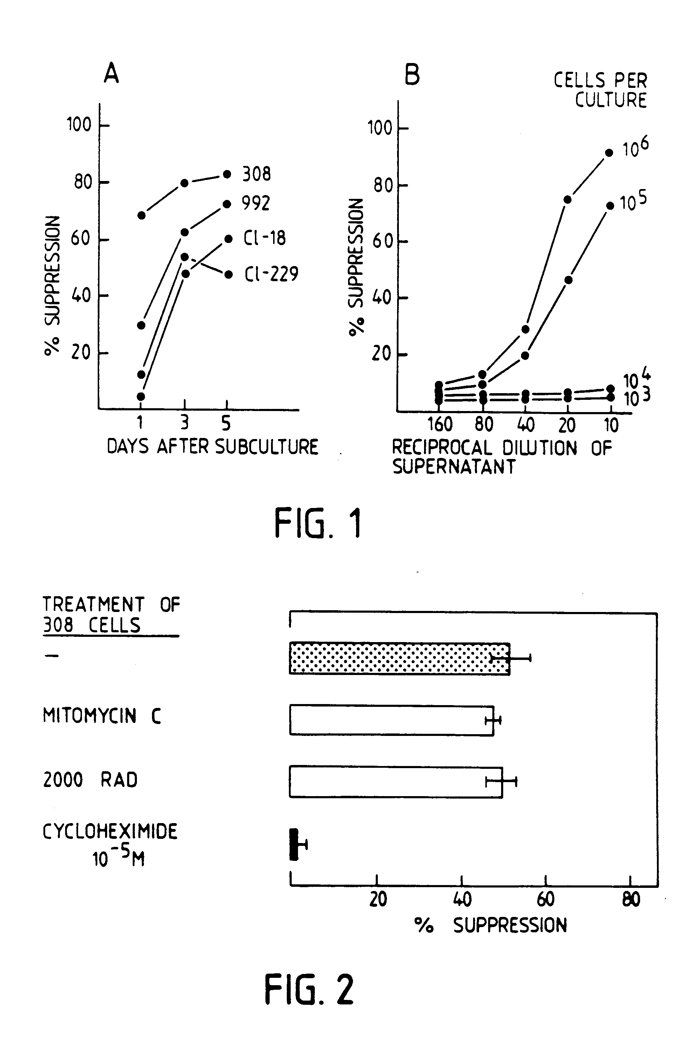

The results are shown in Figures 1A and B as percent suppression compared with the Con A response of thymocytes treated with a medium control.

For stadarisation purposes % suppression can be plotted to give a standard curve from which conversion may be made to units of G-TsF activity.

In Figure 1A the SN of the four glioblastoma cell lines used were harvested after 1, 3 and 5 days and tested at a final dilution of 1/10 on the thymocytes. In Figure 1B the testing took place on SN of 308 cells harvested after culture for 5 days at different seeding densities and final dilutions. In the absence of glioblastoma SN or medium control the background count was 483± 229 and the Con A response 76.797±6.631 cpm.

The magnitude of inhibitory potency of the SN can be seen to be dependent on time of culture and cell density.

In selected experiments on metabolic inhibition using 308 cells the production of SN was performed with irradiated (2000 R) or mitomycin C-treated (50 $\mu$g/ml, 30 min. 37°C) glioblastoma cells. Alternatively cycloheximide ($10^{-5}$M) was added to glioblastoma cells at the beginning of the 72-hour culture; cycloheximide was also added to control SN collected after 72 hours. These SN were ultrafiltrated three times before testing to remove CH.

The results of these experiments are summarised in Figure 2, where the SN of $10^6$ cells cultured over 5 days were employed. As a CH control the SN of CH treated 308 cells and of 308 SN supplemented with CH after collection were ultrafiltrated.

In a further control, experiments were carried out on four established human tumor cell lines unrelated to glioblastoma cells. These results are summarised in Table 1.

### TABLE I

### Effects of SN from various tumor cell lines on lymphocyte blastogenesis

| Type of Malignancy [a] | Designation | Con A Response [b] (cpm $\pm$ SD) | % Suppression |
|---|---|---|---|
| Neuroblastoma | SR.N.SH | $52.123 \pm 1.817$ | 6.0 |
| Neuroblastoma | IMR.32 | $50.041 \pm 2.751$ | 9.8 |
| Melamoma | Me.43 | $58.382 \pm 447$ | 0 |
| Rhabdomyosarcoma | RD | $51.720 \pm 904$ | 6.8 |

[a] Tumor cells were cultured at a seeding density of $10^6$ cells/ml for 5 days.

In the presence of Con A, thymocytes were incubated with 1/10 dilution of tumor cell SN or a control culture medium for the tumor cell SN. In the presence of medium control the background count of thymocytes was $432 \pm 112$ and the Con A response was $55441 \pm 4817$.

It will be noted that the release of immunosuppressive factors does not appear to be a general property of cultured tumor cells.

Analysis of the L-arginine content in 308 SN revealed that inhibition is not due to arginine degradation in the medium by an arginase release by 308 cells. Furthermore, the 308-mediated inhibition could not be explained by the production of interferon as no antiviral activity was detected in 308 SN.

Example 7 Effect of glioblastoma SN on B lymphocytes and non-lymphoid cells

Mouse fibroblasts (A9, 3T3, LS cell lines) were cultured in DMEM supplemented with L-glutamine (300 ug/ml) and 10% FCS: mouse neuroblasts (Neuro 2A, NB4, 1A3 cell lines) were plated in Earle's minimum essential medium with 10% FCS. L-glutamine (300 ug/ml), and 1% nonessential amino acids (100x). Fibroblasts and neuroblasts ($10^4$ cells/well) were incubated in 0.2 ml of medium for 72 hours. For the final 6 hours, the cells were pulsed with 1.2 $\mu$Ci of $^3$H-Tdr. Thereafter, the medium was aspirated from each well, replaced with trypsin-EDTA. and incubated (37°C, 10 min). Finally, the cells were harvested with the use of an automated harvester.

The data in Table II indicate that when compared with its effect on thymocytes, the 308 SN was less inhibitory on B cell proliferation: the percentage of inhibition of the LPS-induced stimulation of spleen cells was 29%, whereas the corresponding value for the myeloma cell line X63-Ag8 was 21%.

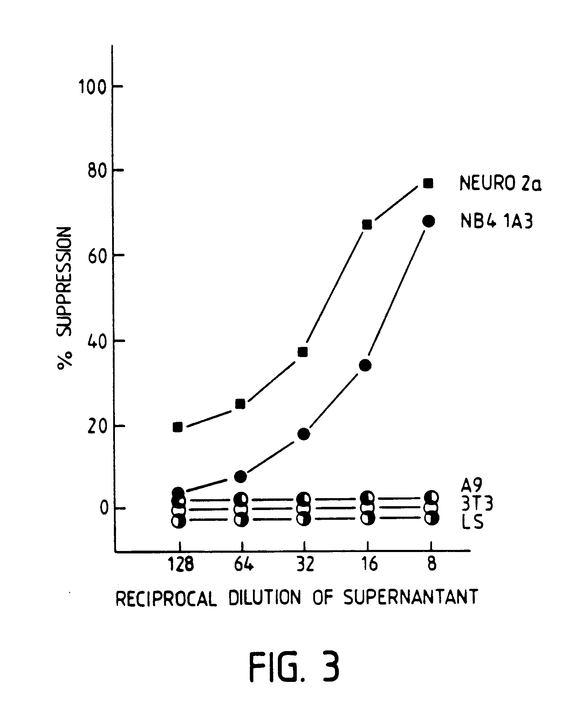

There was no difference in thymidine uptake between fibroblast cultures containing 308 SN and those being supplemented with a medium control. This was true for all three fibroblast cell lines tested. In contrast, the glioblastoma SN significantly suppressed the growth of the two neuroblast cell lines as measured by the uptake of $^3$H-Tdr (Fig.3, Table II) and by counting the number of cells at termination of the 72 hr cultures. Twenty-four, 48 and 72 hr after culturing 1 x $10^4$ Neuro 2A cells/well, the cell numbers x $10^4$/well of quadruplicate cultures were in the presence of the 308 SN:$0.98\pm 0.17$ (24 hr),$1.80\pm 0.36$ (48 hr), and $4.3\pm0.44$ (72 hr), whereas the corresponding values for the medium controls were $0.88\pm0.05$, $2.55\pm0.06$, and $6.08\pm0.40$, respectively.

7

TABLE II

| Effect of 308 SN on lymphoid and nonlymphoid cells | | | | | |
|---|---|---|---|---|---|
| | | | | | $^3$H-Tdr Uptake(cpm) |
| Cell Type | Description or Strain | Stimulant | Medium control | 308 SN[a] | % Suppression |
| Thymocytes | C3H/Hej | Con A | 66.709 ± 3.104 | 8.323 ± 235 | 88 |
| T cell lymphoma | EL-4 | - | 137.960 ± 25.173 | 164.487 ± 6.253 | 0 |
| Spleen cells | DBA | LPS[b] | 72.442 ± 3.886 | 50.990 ± 2.184 | 29 |
| Myeloma cells | X63-Ag8[b] | - | 117.090 ± 2.597 | 92.580 ± 6.351 | 21 |
| Neuroblasts [c] | NB41A3 | - | 48.073 ± 1.756 | 15.504 ± 833 | 68 |
| Neuroblasts | Neuro 2A | - | 250.807 ± 19.700 | 62.408 ± 6.108 | 76 |
| Fibroblasts[c] | A9 | - | 78.591 ± 11.029 | 78.323 ± 10.444 | 0 |
| Fibroblasts | 3T3 | - | 117.316 ± 8.484 | 117.011 ± 4.026 | 0 |
| Fibroblasts | L929 | - | 84.395 ± 6.189 | 92.391 ± 5.674 | 0 |

[a] All assays were performed with the same 308 SN added to give a final dilution of 1/10.

[b] After lysis of erythrocytes 6 x 10$^5$ spleen cells were stimulated with lipopolysaccharide (LPS.E.coli 0127 B8, Difco Lab.). The mouse X63-Ag8 myeloma cells were cultured at 10$^4$ cells/well in DMEM supplemented with 10% FCS.

[c] See legend of Figure 3.

In Figure 3 the SN of 10$^6$ glioblastoma cells (308) cultured for 5 days were employed together with a medium control.

Example 8 - Mechanisms of glioblastoma SN-induced inhibition of T-cell activation

Thymocytes were treated with 308 SN (final dilution of 1/10) in the presence of PHA (0.5 ug/well) and various dilutions (1/10 in 1/160) of IL 1 or IL 2. In the absence of interleukins the background count was 209 ±84 and the PHA response was 3074±425.

The results are summarised in Table III.

TABLE III

| Effect of 308 SN on thymocytes stimulated with PHA and various doses of IL 1 or IL 2 | | | | |
|---|---|---|---|---|
| Stimulant [a] | | $^3$H-Tdr Uptake (cpm) | | % Suppression |
| | | Medium control | 308 SN | |
| PHA + IL 1[b] | 1/10 | 76.342 ± 5.978 | 12.104 ± 618 | 84.2 |
| | 1/20 | 69.831 ± 5.150 | 13.423 ± 212 | 81.8 |
| | 1/40 | 42.143 ± 1.706 | 9.061 ± 842 | 78.5 |
| | 1/80 | 18.407 ± 128 | 2.648 ± 221 | 85.7 |
| PHA + IL 2[b] | 1/10 | 84.457 ± 3.621 | 13.551 ± 817 | 84.0 |
| | 1/20 | 84.882 ± 4.185 | 15.492 ± 1.005 | 81.8 |
| | 1/40 | 76.537 ± 2.404 | 11.250 ± 1.423 | 85.4 |
| | 1/80 | 43.244 ± 1.753 | 5.973 ± 175 | 86.2 |
| | 1/160 | 26.505 ± 1.044 | 3.182 ± 3.279 | 88.0 |

[a] Thymocytes were treated with 308 SN (final dilution of 1/10) in the presence of PHA (0.5 μg/well) and various dilutions (1/10 to 1/160) of IL 1 or IL 2. In the absence of interleukins the background count was 209 ± 84 and the PHA response was 3074 ± 425.

[b] The IL 1 preparation used was prepared from LPS-stimulated peritoneal cells of BALB/c mice; the IL 2 was prepared by stimulation of BALB/c spleen cells with Con A-Sepharose as described previously (17).

These results show that inhibition of the Con A response of thymocytes apparently results from influences on late events of the T cell activation cascade, because the 308 SN inhibited the proliferative response of thymocytes stimulated with PHA in the presence of IL 1 or IL 2. The degree of 308 SN-induced suppression of proliferation was similar at various concentrations of IL 1 or IL 2 employed.

Example 9 - Effect of SN-308 on cloned T-cell lines

Proliferation of H-2$^b$-restricted hapten (N-iodoacetyl-N-(5-sulfonic-1-naphthyl) ethylene diamine [AED])-specific cloned cytotoxic T-cells (CTL) (10) was assessed in culture medium consisting of Iscove's modified DMEM supplemented with 10% FCS, 1 x 10$^5$ M 2-ME, antibiotics and 10% 10% (v/v) of a 24-hr spleen cell Con A SN (IL 2 SN). The CTL (10$^4$/well) were cultured alone or together with 10$^6$ irradiated (2000 rad) haptenated spleen cells (H-2$^b$) as stimulators. Various dilutions of SN of 308 glioblastoma cells or of a control medium were added to the mixture of the CTL and stimulators cultured in culture medium for 72 hr. Sixteen hours before harvest, each well was pulsed with 1.2 μCi of $^3$H-Tdr.

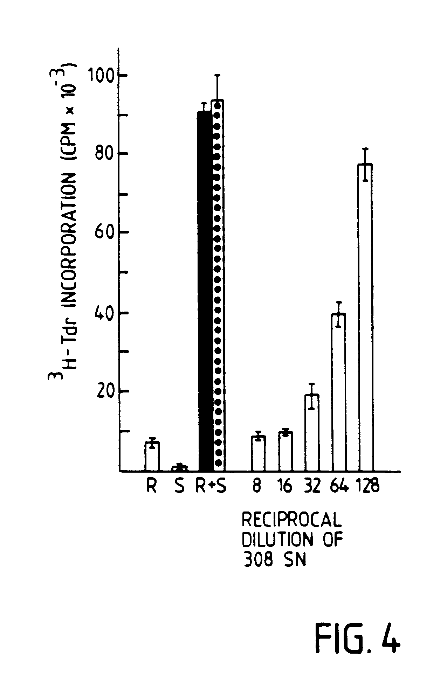

The results are shown in figure 4. Hapten - specific cloned CTL were cultured (at 10$^4$ cells) alone (R) or together with 10$^6$ irradiated (2000 rad) AED-haptenated spleen cells as stimulators (S) (black bar). Various dilutions (1/8 to 1/128) of 308 glioblastoma SN or a 1/8 dilution of control medium

( [ ⋯⋯ ] )

were added to the mixture of CTL and cultured for 72 hr. Data are given as counts per minute of $^3$H-Tdr incorporation. Figure 4 shows that cells of an H-2-restricted, hapten-specific T-cell line proliferated in the presence of IL 2 and haptenated, irradiated spleen cells as stimulator cells. This proliferation was completely blocked by adding the glioblastoma-produced factor.

From the experiments outlined above, the inhibition of T cell growth by glioblastoma cell-derived factors could be due to their direct interference with IL 2-triggered events leading to T cell proliferation. This possibility is supported further by the finding that the 308 SN had no effect on the growth of a thymoma cell line that grows independently of IL 2 (Table II).

EXAMPLE 10:

The release of analogous inhibitory factors by the glioblastoma cells in vivo was demonstrated by testing the ultrafiltrated cyst fluid of the patient from whom the 308 cells originated. The cyst fluid (final dilution 1/20), ultrafiltrated on YMB membranes, induced a 28% inhibition of the IL 2-induced proliferation of OVA T cells, whereas 12 control sera had no effect.

EXAMPLE 11:

Effect of glioblastoma SN on the induction of cytotoxic T-cells

Anti-H-$2^b$-specific CTL were induced by stimulation of 12 x $10^6$ B10.D2 ( H-$2^d$) spleen cells with 30 x $10^6$ irradiated (2000 R) C57B1/6 ( H-$2^b$) stimulator spleen cells in 6 ml Iscove's modified DMEM containing 10 % FCS. 1 x $10^{-5}$ M 2-ME, and antibiotics (mixed lymphocyte culture (MLC) medium). After 6 days the specific cytotoxic activity of the CTL was tested in a 3-hr $^{51}$Cr-release assay in 0.2 ml cultures containing $10^4$ $^{51}$Cr-labelled target cells (EL-4 (H-$2^b$), P815 (H -$2^d$). Yac-1 ( H-$2^d$)) at different target ratios as described in 11. The cytotoxic effect of the cells cultured over 6 days in MLC medium was compared with the effects of cells cultured in MLC medium being supplemented with medium control or 308 SN.

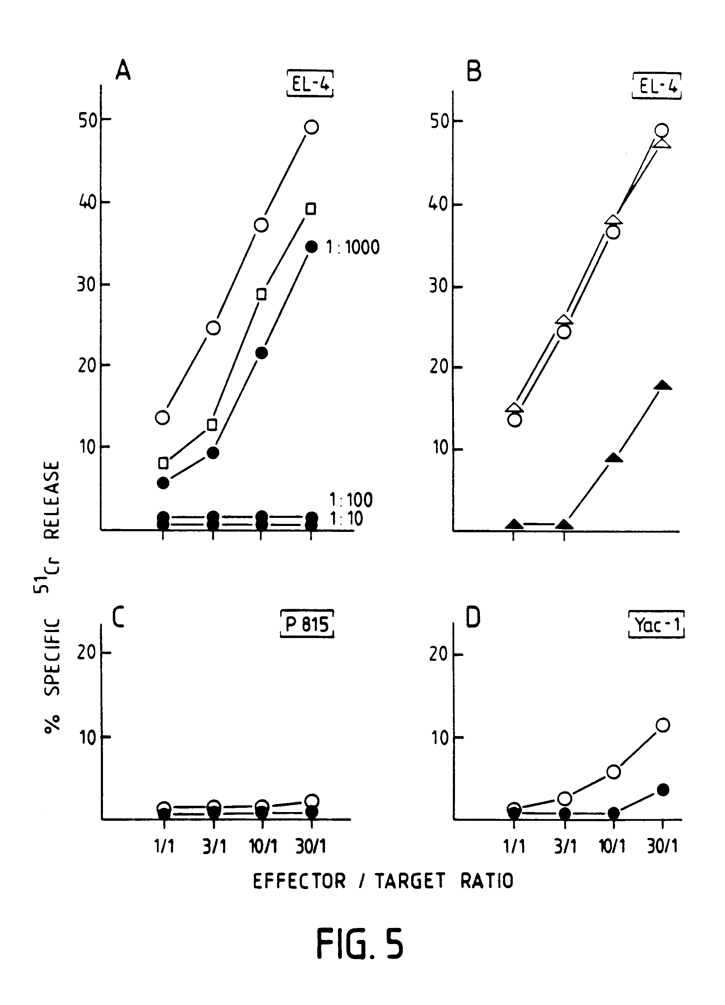

The results are summarized in Fig. 5A-D. Legend Fig. 5. The cytotoxic effect of the cells cultured over 6 days in MLC medium (0-0) was compared with the effects of cells cultured in MLC medium being supplemented with a) medium control (final dilution 1/10)

$$(\square\text{————}\square) ,$$

b) 308 SN

$$(\bullet\text{————}\bullet)$$

diluted 1/10 (A-D). 1/100 or 1/1000 (A) or with c) Sephacryl S-200 column fractions that were known to inhibit

$$(\blacktriangle\text{————}\blacktriangle)$$

or to have no effect

$$(\triangle\text{————}\triangle)$$

on the IL 2-dependent proliferation of OVA T cells. The fractions to be assayed were concentrated 10 x on YMB membranes, and the concentrated fractions were added to MLC medium to give a final dilution of 1/10. Results are given as percent specific $^{51}$Cr release. Test duration 3 hr. Mean values of three samples.

The data indicate a 308 SN induced inhibition of the generation of CTL. The inhibitory effect is mediated by factors with an apparent molecular weight of 97,000.

(The preparation and testing of Sephacryl S-200 column fractions is described below).

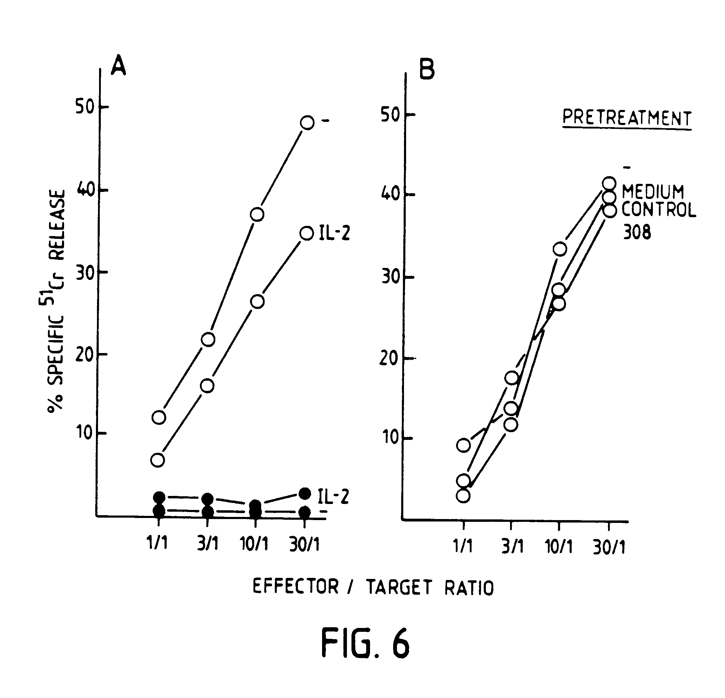

As shown in figure 6 the addition of IL 2 together with the 308 SN factor did not restore the generation of CTL. A 2-hr preincubation of mature CTL with 308 SN had no influence on the function of CTL on their targets.

In Fig. 6 A during the entire culture period of 6 days, IL 2 (final concentration of 10 % Con A SN) was present in MLC treated with 308 SN (1/10 diluted)

$$(\bullet\text{————}\bullet)$$

or medium control

$(\bigcirc\!\!-\!\!\bigcirc).$

After 6 days the CTL activity was tested on EL-4 cells. In B. before testing on EL-4 cells, CTL generated over 6 days in culture medium were preincubated for 2 hr in medium control or 308 SN (1/4 diluted).

EXAMPLE 12:

Biochemical characterisation of the glioblastoma SN

The 308 glioblastoma SN obtained from 308 cells cultured in serum free conditions over 96hr was concentrated 30-fold by ultra-filtration on YM-10 (Amicon) membrane, and 1.5 ml of concentrated SN was applied on a Sephacryl S-200 (column size 2.5 x 85 cm) at 4°C as described (9). The eluate collected in 2.5 ml fractions was monitored for adsorbance at 280 nm and was tested on fibroblasts (L 929) and neuroblasts (Neuro 2 A) as described above. In addition the fractions were tested on OVA cells in the presence of IL2 and on thymocytes stimulated with PHA. The column, in a parallel run, was calibrated with $\gamma$-globulin ($\gamma$-Glob. m.w. 150.000). bovine serum albumin (BSA. m.w. 67.000), ovalbumin (OVA, m.w. 45.000), $\alpha$-chymotrypsinogen (CHY. m.w. 25.000), and cytochrome c (CYT C. m.w. 12.400).

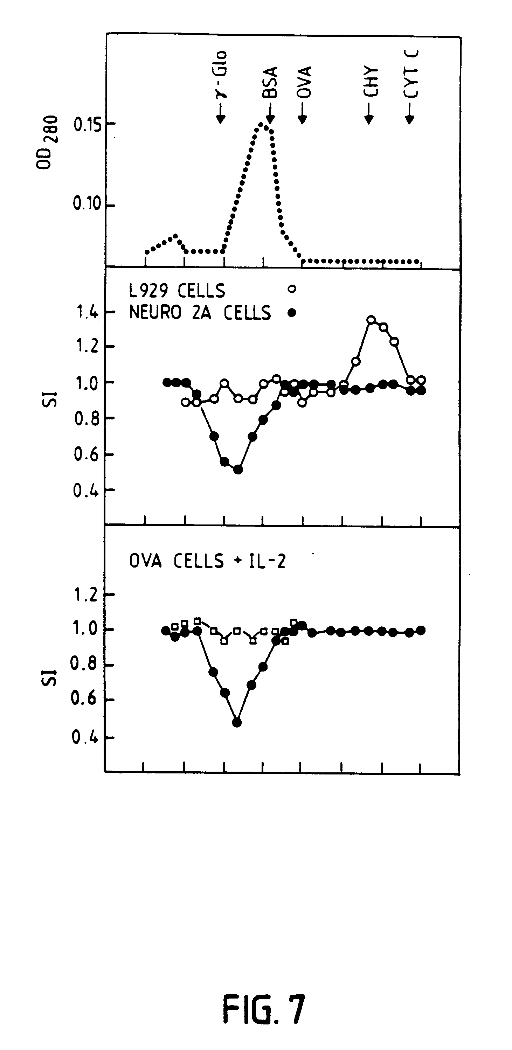

These results are summarized in figure 7 whereby the upper table shows the calibration run. Results are given as stimulation index (SI)comparing proliferation of cells treated with column fractions to cells treated with medium control. Results of serum-free control medium fractionated on a Sephacryl S-200 column are shown on OVA cells treated with IL 2

$(\square\!\!-\!\!\square).$

The peak of the inhibitory activity on neuroblasts and OVA cells was eluted from the column at the identical position, with an apparent m.w. of 97.000 (Fig. 7). Sephacryl S-200 fractions of a medium control consisting of DMEM (Fig. 7) and a medium control supplemented with 10 % FCS (data not shown) had no inhibitory effects on the growth of OVA cells stimulated with IL 2. Furthermore, no inhibitory effect was observed when testing 308 SN fractions on fibroblasts.

As evidenced in the previous examples the immunosuppressant factor according to the invention is indicated for use in the treatment of diseases and conditions where suppression of the body's immune response is desired. Thus use in connection with transplant operations to prevent rejection is indicated. Examples of diseases where suppressions body's immune systems is indicated include the so-called auto-immune diseases.

For such use as daily dosage of about 10 to 2000 $\mu$g e.g. 200 to 1000 $\mu$g per patient is typically indicated.

The factor according to the invention can be employed in mixed form as purified supernatant from appropriate glioblastoma cell lines or in fractionated and purified form as hereinbefore described.

It can be got up in suitable form for administration in a manner conventional for substances of this nature in accordance with the condition to be treated. It can thus be injected intravenously in a suitable pharmacological carrier.

**Claims**

1. An immunosuppressant factor isolated from human glioblastoma cells which;

   aa) inhibits the incorporation of tritiated thymidine into murine thymocytes stimulated with Concanavalin A or phytohaemaglutinin in the presence of IL-2;
   bb) inhibits the proliferation of IL-2 dependent T cell clones;
   cc) suppresses the growth of neuroblasts but not fibroblasts;
   dd) inhibits the generation of cytotoxic T cells in the allogenic mixed lymphocyte reaction;
   ee) inhibits the proliferation of hapten-specific cytotoxic T cells in the presence of haptenated stimulator;
   ff) inhibits the proliferative response of thymocytes to concanavalin A and
   hh) is sensitive to tryptic proteolysis.

2. A method of producing an immunosuppressant factor according to claim 1 which comprises concentrating the supernatant of a human glioblastoma cell line by ultrafiltration and subjecting same to sequential chromatography on Blue Affigel, hydroxyapatite and gel filtration.

**Revendications**

1. Un facteur immunosuppresseur isolé de cellules de glioblastome humain, qui
   aa) inhibe l'incorporation de thymidine tritiée dans les thymocytes murins stimulés par la concanavaline A ou la phytohémagglutinine en présence de IL-2,
   bb) inhibe la prolifération de clones de cellules T dépendantes de IL-2,
   cc) empêche la croissance des neuroblastes mais pas celle des fibroblastes,
   dd) inhibe la génération de cellules T cytotoxiques dans la réaction lymphocytaire mixte allogénique,
   ee) inhibe la prolifération de cellules T cytotoxiques spécifiques d'haptène en présence d'un stimulant combiné à l'haptène,
   ff) inhibe la réponse proliférative de thymocytes à la concanavaline A et
   hh) est sensible à la protéolyse par la trysine.

2. Un procédé de préparation d'un facteur immunosuppresseur selon la revendication 1, caractérisé en ce qu'on concentre le surnageant d'une lignée cellulaire de glioblastome humain par ultrafiltration et on le soumet à une chromatographie séquentielle sur Blue Affigel, hydroxyapatite et filtration sur gel.

**Patentansprüche**

1. Ein aus menschlichen Glioblastoma Zellen isolierter Immunsuppressionsfaktor, der
   aa) den Einbau von mit Tritium markierten Thymidin in Maus-Thymozyten hemmt, die mit Concanavalin A oder Phytohämagglutinin in Gegenwart von Il-2 stimuliert werden;
   bb) die Proliferation von IL-2 abhängigen T Zellklonen hemmt;
   cc) das Wachstum von Neuroblasten, aber nicht von Fibroblasten unterdrückt;
   dd) die Bildung von zytotoxischen T Zellen in der allogenen gemischten Lymphozytenreaktion hemmt;
   ee) die Proliferation von haptenspezifischen zytotoxischen T Zellen in Gegenwart von mit Haptenen versehenem Stimulator hemmt;
   ff) die proliferative Antwort von Thymozyten auf Concanavalin A hemmt und
   hh) empfindlich gegenüber tryptischer Proteolyse ist.

2. Ein Verfahren, einen Immunsuppressionsfaktor nach Anspruch 1 herzustellen, welches Verfahren ein Konzentrieren des Überstandes von einer humanen Glioblastoma Zell-Linie durch Ultrafiltration und ein Unterziehen desselben einer sequentiellen Chromatographie auf "Blue Affigel", einer Hydroxyapatit- und einer Gel-Filtration umfaßt.

FIG. 1

FIG. 2

FIG. 3

FIG. 4

FIG. 5

FIG. 6

FIG. 7

FIG. 8

FIG. 9

FIG. 10

TABLE IV  EVALUATION OF ADSORBENTS FOR CHROMATOGRAPHY OF T-CELL SUPPRESSOR FACTOR

| Adsorbent | G-TsF activity | | Total protein | | Adsorption | Desorption | pH |
|---|---|---|---|---|---|---|---|
| | free [a] (Units) | bound [b] (Units) | free [a] (mg) | bound [b] (mg) | | | |
| Hydroxyapatite | 16 | 74 | 0.2 | 1.7 | 0.01 M Tris-HCl 0.5 M NaCl | 0.4 M Na-phos-phate | 7.5 |
| Blue-Affigel | 23 | 61 | 1.2 | 1.0 | 0.01 M Tris-HCl | 2.0 M NaCl | 7.5 |
| Phenyl-Sepharose | 32 | 51 | 0.9 | 0.7 | 0.25 M Na-phos-phate | 80% ethylene glycol | 7.5 |
| DEAE-Cellulose | 29 | 67 | 0.1 | 2.0 | 0.01 M Tris HCl | 1.0 M NaCl | 8.0 |
| DEAE-Cellulose | 45 | 43 | 0.2 | 1.8 | 0.01 M Na-Hepes | 1.0 M NaCl | 6.4 |
| Heparin-Affigel | 55 | 50 | 1.8 | 0.8 | 0.01 M Tris-HCl | 1.0 M NaCl | 7.5 |
| CM-Cellulose ConA-Sepharose Protein A-Sepharose | > 80 | < 20 | >2 | <0.5 | | | |

a) After having equilibrated each adsorbent with 100 units of G-TsF (2.5 mg total protein) under the adsorption conditions listed here, the G-TsF activity and total protein remaining in solution were measured.

b) G-TsF activity and total protein bound to the column were determined after desorption under the conditions listed here.

EP 0 159 289 B1

TABLE V    SUMMARY OF G-TsF PURIFICATION

| Step | | Volume (ml) | G-TsF activity[a] (units) | yeald (%) | Protein[b] (mg) | Specific activity (units/mg) | Purification factor |
|---|---|---|---|---|---|---|---|
| | Crude 308 supernatant | 150 | 13800 | – | 360 | 38 | – |
| 1 | Amicon-concentrate | 50 | 13500 | 98 | 350 | 38 | 1 |
| 2 | Blue-Affigel 1.0 - 1.5 M NaCl eluate | 70 | 7600 | 55 | 42 | 180 | 5 |
| 3 | Hydroxyapatite, 0.25-0.35 M Na phosphate eluate | 22 | 6800 | 49 | 0.10 | 70000 | 1900 |
| 4 | Ultrogel AcA 54 | 75 | 6250 | 45 | 0.08 | 75000 | 2000 |

[a]    Determined by ConA/thymocyte assay (see Material and Methods)

[b]    Determined by amidoblack assay

EP 0 159 289 B1

TABLE VI  INACTIVATION OF G-TsF BY TRYPTIC PROTEOLYSIS

| Treatment | G-TsF activity | |
|---|---|---|
| | Units/ml | % of control |
| Control [a] | 164 | 100 |
| TPCK-trypsin 40 μg/ml, 1 hr, 37° C | 42 | 26 |
| Immobilized RNase, 1 hr, 37° C | 62 | 38 |
| Immobilized Trypsin, 1 hr, 37° C | 4 | 2 |

[a] TPCK-trypsin added at the end of the incubation period

EP 0 159 289 B1